Europäisches Patentamt

European Patent Office

Office européen des brevets

(11) Veröffentlichungsnummer: **0 285 826 B1**

(19)

(12) # EUROPÄISCHE PATENTSCHRIFT

(45) Veröffentlichungstag der Patentschrift: **15.04.92**

(21) Anmeldenummer: **88103477.1**

(22) Anmeldetag: **31.03.88**

(51) Int. Cl.⁵: **A61L 27/00**, A61K 6/06

(54) **Implantat mit bioaktivem Überzug.**

(30) Priorität: **04.04.87 DE 3711426**

(43) Veröffentlichungstag der Anmeldung:
**12.10.88 Patentblatt 88/41**

(45) Bekanntmachung des Hinweises auf die
Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**EP-A- 0 042 783**
**EP-A- 0 202 908**
**EP-A- 0 219 078**
**DE-A- 2 008 010**

**SPRECHSAAL, Band 119, Nr. 7, Juli 1986,
Seiten 566-569,1131-1134, Muller und
Schmidt, Coburg, DE; A.O. NEBELUNG et al.:
"Über die Eigenschaften von Calciumphosphaten im Hinblick auf ihre Verwendung in
der Biokeramik"**

(73) Patentinhaber: **BK LADENBURG GmbH, Gesellschaft für chemische Erzeugnisse
Dr.-Albert-Reimann-Strasse 2
W-6802 Ladenburg(DE)**

Patentinhaber: **MTU MOTOREN- UND
TURBINEN-UNION MÜNCHEN GMBH
Dachauer Strasse 665 Postfach 50 06 40
W-8000 München 50(DE)**

(72) Erfinder: **Adam, Peter, Dr.
Hackenländerstrasse 9
W-8060 Dachau(DE)**
Erfinder: **Nebelung, Adolf, Dr.
Leipziger Strasse 10
W-6834 Ketsch(DE)**
Erfinder: **Vogt, Michael
Reisener Weg 27
W-6943 Moerlenbach(DE)**

(74) Vertreter: **Grussdorf, Jürgen, Dr. et al
Patentanwälte Zellentin & Partner Rubensstrasse 30
W-6700 Ludwigshafen(DE)**

**Beschreibung**

Die Erfindung betrifft ein Verfahren Zur Beschichtung metallischer oder keramischer Knochenersatzteile oder Zahnersatzteile mit bioaktiven Substanzen.

Die bioaktiven Substanzen sollen dabei behilflich sein, das Anwachsen der Implantate an das Knochengerüst wenigstens teilweise im Körper zu fördern. Durch dieses Anwachsen soll vermieden werden, daß ein Implantat nur rein mechanisch in dem Knochen verankert ist. Dabei darf das Implantat keiner Abstoßungsreaktion unterliegen, d. h. es soll gewebefreundlich sein und die Verbindung zum Knochen muß belastbar sein, was bedeutet, daß das Implantat für eine ausreichende Zeit in dem Körper verbleiben kann.

Nach derzeitigem Kenntnisstand eignen sich als bioaktive Substanzen vor allem Tricalciumphosphat (TCP) und Hydroxylapatit (HA), wobei HA bevorzugt ist.

Es sind bereits Implantate, die teilweise eine Schicht mit Teilchen aus Calciumphosphat tragen, bekannt, z. B. aus der deutschen Patentschrift 27 33 394, deutschen Auslegeschrift 28 24 118, deutschen Patentschrift 30 34 086. In der deutschen Offenlegungsschrift 33 16 801 ist als bioaktives Material sowohl Hydroxylapatit als auch Tricalciumphosphat erwähnt und gleiches gilt für die deutsche Offenlegungsschrift 34 14 992. In beiden letztgenannten Schriften sind jedoch besondere Maßnahmen angeführt, die ein festes und sicheres Verwachsen des Implantates mit dem umgebenden Gewebe gewährleisten.

Das Aufbringen von dünnen Schichten aus keramischem Material auf Metalloberflächen ist im Prinzip bekannt (vgl. z. B. P. Adam, Fortschr. Zahnärztl. Implant. 1, S. 41-46 (1984)).

Aus der Zeitschrift "Sprechsaal", Vol. 119, No. 12, 1986, 1131 bis 1134 ist weiterhin bekannt, daß $\alpha$- und/oder $\beta$-Tricalciumphosphat sich in heißem Wasser in Hydroxylapatit umlagern. Es wird dabei angeregt, für die Verwendung von Calciumphosphaten als Zwischenschicht zwischen Implantaten und Geweben eine Grundschicht von in Wasser praktisch unlöslichen Dicalciumphosphaten aufzubringen, welche durch Behandlung von Wasser in ihrer Oberfläche in Hydroxylapatit umgewandelt wird. Entsprechende Schichten lassen sich nur unter Schwierigkeiten und nicht reproduzierbar und stabil herstellen.

Will man auf dem Implantat gut haftende Schichten erzeugen, die auch gut anwachsen und Lasten tragen können, so hat es sich bewährt, neben der obigen Auswahl von geeigneten Materialien, die Herstellungs- und Sinterbedingungen einer solchen Schicht zu kontrollieren. Dabei kann auch die Porosität, z. B. eines gesinterten Apatitmaterials, geregelt werden. Bei den zum Sintern erforderlichen Temperaturen tritt eine partielle Zersetzung des HA unter Abgabe seines Konstitutionswassers in Tricalciumphosphat und Tetracalciumphosphat ein, so daß kein reines Hydroxylapatit mehr vorliegt und die entstandenen Schichten für das Anwachsen des Implantats nicht mehr optimal sind.

In der DE-A 2008010 ist ein Verfahren zur Herstellung von Knochen- und Zahnersatzteilen durch Heißaufspritzen eines "Eutektoids aus Tricalciumphosphat und Tetracalciumphosphat" auf entsprechende Implantatkerne und Überführen der dabei erhaltenen Schicht in Hydroxylapatit durch Glühen der Körper bei 1000°C in einer Wasserdampf-Atmosphäre beschrieben. Dabei wird in einer Festphasenreaktion aus Tricalciumphosphat und Tetracalciumphosphat unter Zutritt von Wasserdampf Hydroxylapatit gebildet, wozu offensichtlich sehr hohe Temperaturen notwendig sind, die eine Verwendung dieses Verfahrens bei thermisch empfindlichen Knochen- und Zahnersatzteilen unmöglich machen. Wieweit sich dabei reiner Hydroxylapatit bildet, geht aus der Literaturstelle nicht hervor.

Aus der EP-A 0042783 ist ein Verfahren zur Herstellung von Hydroxylapatitschichten auf Implantaten bekannt, bei denen verschiedene Calciumphosphate in einer Wasserdampf-Atmosphäre auf die Implantatkerne bei Temperaturen von 200 - 600°C aufgesprüht werden, wobei sich direkt eine Schicht von Hydroxylapatit ablagert. Gemäß den Beispielen ist diese Schicht jedoch kein reiner Hydroxylapatit, sondern überwiegend mit anderen Phosphaten verunreinigt. Auch dieses Verfahren kann nicht angewendet werden, um thermisch empfindliche Knochen- und Zahnersatzteile zu beschichten, da diese bei den hohen Temperaturen zerstört würden.

Aufgabe der Erfindung ist die Herstellung einer reinen, nicht porösen Hydroxylapatitschicht.

Die Lösung dieser Aufgabe besteht in der Herstellung einer chemisch reinen Schicht aus Tricalciumphosphat auf metallischen oder nicht-metallischen Körpern für Implantate und Umwandlung in eine Schicht aus Hydroxylapatit durch Reaktion mit flüssigem Wasser bei erhöhter Temperatur.

Weitere Merkmale der Erfindung sind der Beschreibung, dem Ausführungsbeispiel und den Ansprüchen zu entnehmen.

Zur Erfindung gehören auch Kombinationen des Umwandlungsverfahrens mit Schichtherstellungsverfahren sowie die Anwendung solcher Schichten auf Implantaten, insbesondere Dentalimplantaten oder endorsalen Implantaten, wie Hüftgelenksknochen, Kniegelenken oder auch Prothesen oder Teilprothesen.

Mit der Erfindung werden vor allem folgende wesentliche Vorteile erzielt:
Die Erfindung zeigt eine Möglichkeit auf, Calciumphosphatschichten bei solch hohen Temperaturen aufzubringen, bei denen der Hydroxylapatit sonst zerfallen würde und dadurch dichte, d.h. wenig poröse Schichten herzustellen. Die in Hydroxylapatit umgewandelten Schichten sind sehr gut gewebeverträglich, sie gestatten eine schnellere Resorption und Einheilung. Auch unter Belastung können die neuen Implantate für eine längere Verweilzeit im Körper bleiben.

Um eine HA-Schicht zu erhalten, wird zunächst reines TCP aufgebracht, z. B. durch thermisches Spritzen, Bedampfen oder Sputtern in einer geeigneten, an sich bekannten Vorrichtung. Dabei entsteht eine ausreichend dichte reine TCP-Schicht ohne Zerfallsprodukte. Entsteht durch hohe Abkühlgeschwindigkeiten $\alpha$-TCP (Hochtemperaturmodifikation), so läßt sich daraus durch nachfolgende Wärmebehandlung $\beta$-TCP herstellen.

Die so erhaltene $\beta$- oder $\alpha$-TCP-Schicht auf dem Implantat wird vollständig in HA umgewandelt, in dem die Implantate in flüssigem Wasser erhitzt werden. Die Wassertemperatur wird nach den apparativen Möglichkeiten eingestellt. Die Reaktionsgeschwindigkeit der Umwandlung der TCP-Schicht in eine HA-Schicht ist eine Funktion der Temperatur. Je höher die Temperatur gewählt wird, desto schneller erfolgt die Umwandlung. Allerdings ist zu berücksichtigen, daß auch der in Wasser gelöste Anteil mit steigender Temperatur steigt. Der zweckmäßigste Temperaturbereich ist 60 bis 90°C.

Ausführungsbespiel

A. Herstellung der Beschichtungen

Zylindrische Normkörper aus Titan mit Längen von 8,0 (Typ I) bzw. 12,5 cm (Typ II) und einem Durchmesser von 4,0 cm, d. h. einer Oberfläche von 113 bzw. 170 cm$^2$ werden sorgfältig poliert, um die oberflächliche Oxidschicht zu entfernen, und mit verdünnter Salzsäure und Alkohol gereinigt. Es wird angenommen, daß auch nach dieser Behandlung noch eine TiO$_2$-Schicht von 0,01-0,02 $\mu$m die Oberfläche bedeckt.

Auf diese Körper wird nach der Methode des Plasma-Spritzens in mehreren Schichten $\beta$-Tricalciumphosphat aufgebracht, bis eine ca. 180 $\mu$m starke, feste, gleichmäßige Schicht entstanden ist. Aus den Röntgendifraktogrammen ergibt sich, daß die gebildete Schicht aus der reinen Hochtemperaturform, d. h. $\alpha$-TCP besteht. Stereomikroskopische Untersuchungen zeigen, daß die Schicht glatt und ohne Risse und Poren ist.

Durch eine längere Wärmebehandlung läßt sich das $\alpha$-TCP in $\beta$-TCP zurückverwandeln. Da die Löslichkeit beider Phasen ungefähr gleich ist, wurde mit der ursprünglich entstandenen $\alpha$-TCP-

Schicht weitergearbeitet.

B. Umwandlung der TCP-Schicht in HA

In die Papierhülse einer Soxletapparatur (Volumen ca. 70 ml) werden 8 Körper Typ I bzw. 10 Körper Typ II eingebracht und 450 ml destilliertes Wasser im Destillierkolben vorgelegt. Die Zahl der Umläufe betrug 7,5 pro Stunde, die mittlere Temperatur in der Hülse 80°C.

Die Umwandlung von TCP in HA wird an Hand des in der Zeiteinheit in Lösung gehenden Phosphats welches sich in dem Destillierkolben anreichert, verfolgt. Die Phosphatmenge wird dazu in wöchentlichen Abständen photometrisch bestimmt.

Die Fig. 1 und 2 zeigen die Abnahme der TCP-Löslichkeit, wobei nach ca. 4-6 Wochen die praktisch vernachlässigbare Löslichkeit des HA eingestellt ist (ca. 2 mg/1000 mm$^2$ Woche).

Röntgendiffraktogramme des vom Titankörper abgeschabten Beschichtungsmaterials zeigen, daß sich das TCP vollständig in HA umgewandelt hat. Stereomikroskopische Aufnahmen zeigen, daß auch die umgewandelte Schicht noch glatt, ohne Risse und Poren ist.

Der vorstehende Versuch wird wiederholt, wobei die TCP-beschichteten Körper direkt in einer 0,1 molaren Salzsäure gekocht werden. Die Umwandlung in HA wird durch die erhöhte Temperatur und gegebenenfalls durch den Säurezusatz in 10-20 Tagen erreicht.

**Patentansprüche**

1. Verfahren zur Herstellung einer Schicht aus Hydroxylapatit (HA) auf metallischen und nicht-metallischen Körpern für Implantate durch Aufbringen einer Schicht aus Calciumphosphaten und Umwandlung derselben in HA bei hohen Temperaturen, **dadurch gekennzeichnet,** daß eine Schicht aus $\alpha$-oder $\beta$-Tricalciumphosphat (TCP) aufgebracht und vollständig in reines HA durch Reaktion mit flüssigem Wasser bei erhöhten Temperaturen umgewandelt wird.

2. Verfahren nach Anspruch 1, dadurch gekennzeichnet, daß die Umwandlung bei Temperaturen von 80-100°C durchgeführt wird.

3. Verfahren nach Anspruch 1 oder 2, dadurch gekennzeichnet, daß die wäßrige Phase einen pH-Wert von 7 bis 2 aufweist.

4. Verfahren nach Anspruch 1-3, dadurch gekennzeichnet, daß die Schicht aus $\alpha$-TCP besteht.

**5.** Verfahren nach Anspruch 1-4, dadurch gekennzeichnet, daß die TCP-Schicht durch thermisches Spritzen, Bedampfen, Sputtern mit α- oder β-TCP und Abwandlungen dieser Verfahren aufgebracht worden ist.

**6.** Implantate erhalten nach einem Verfahren gemäß Ansprüchen 1-5.

## Claims

**1.** Process for the production of a layer of hydroxylapatite (HA) on metallic and non-metallic bodies for implants by application of a layer of calcium phosphates and conversion thereof into HA at high temperatures, characterised in that a layer of α- or β-tricalcium phosphate (TCP) is applied and completely converted into pure HA by reaction with liquid water at elevated temperatures.

**2.** Process according to claim 1, characterised in that the conversion is carried out at temperatures of 80 - 100°C.

**3.** Process according to claim 1 or 2, characterised in that the aqueous phase has a pH value of 7 to 2.

**4.** Process according to claims 1 - 3, characterised in that the layer consists of α-TCP.

**5.** Process according to claims 1 - 4, characterised in that the TCP layer has been applied by thermal spraying, vacuum deposition, sputtering with α- or β-TCP and modifications of these processes.

**6.** Implants obtained by a process according to claims 1 - 5.

## Revendications

**1.** Procédé de fabrication d'une couche d'hydroxyapatite (HA) sur des objets métalliques et non métalliques destinés à des implants, par application d'une couche de phosphates de calcium et conversion de ces derniers en HA à haute température, caractérisé en ce qu'une couche de phosphate tricalcique (TCP) α ou β est appliquée et est entièrement convertie en HA pur par réaction avec de l'eau liquide à haute température.

**2.** Procédé selon la revendication 1, caractérisé en ce que la conversion a lieu à des températures de 80-100°C.

**3.** Procédé selon la revendication 1 ou 2, caractérisé en ce que la phase aqueuse a un pH de 7 à 2.

**4.** Procédé selon les revendications 1 à 3, caractérisé en ce que la couche est constituée de α-TCP.

**5.** Procédé selon les revendications 1 à 4, caractérisé en ce que la couche de TCP est appliquée par pulvérisation thermique, déposition en phase vapeur, pulvérisation cathodique avec du α- ou du β-TCP, et des variantes de ces procé-dés.

**6.** Implants obtenus par un procédé selon les revendications 1 à 5.